# EUROPEAN PATENT APPLICATION

(11) **EP 1 864 653 A2**
(43) Date of publication of application: **12.12.2007**
(21) Application number: 06725817.8
(22) Date of filing: 14.03.2006
(51) Int. Cl.: A61K 9/51, A61K 9/16, A61K 47/30, C08G 63/91, C08B 37/08

(54) **NANOPARTICLES OF CHITOSAN AND POLYETHYLENEGLYCOL AS A SYSTEM FOR THE ADMINISTRATION OF BIOLOGICALLY-ACTIVE MOLECULES**

(30) Priority: 14.03.2005 ES 200500590
(71) Applicant: Advanced in Vitro Cell Technologies, S.L., 08028 Barcelona (ES)
(72) Inventor: ALONSO FERNÁNDEZ, José Pabell n de Servicios, E-15782 Santiago De Compostella (ES); JANES, Kevin Pabell n de Servicios, E-15782 Santiago De Compostela (ES); CSABA, Noemi Pabell n de Servicios, E-15782 Santiago De Compostela (ES)
(74) Representative: ABG Patentes, S.L.
(86) International application number: PCT/ES2006/000123
(87) International publication number: WO 2006/097558

(57) **Abstract**

The present invention relates to nanoparticle systems for the release of biologically active molecules formed by the chitosan polymer or its derivatives, chemically modified with polyethylene glycol and crosslinked with a crosslinking agent. These systems are especially useful for pharmaceutical compositions, vaccines and cosmetic formulations.

## Description

### FIELD OF THE INVENTION

The invention is aimed at nanoparticle systems for the release of biologically active molecules. It is specifically aimed at nanoparticle systems formed by an ionically crosslinked chitosan-polyethylene glycol conjugate in which a biologically active molecule can be located, as well as processes for obtaining it.

### STATE OF THE ART

The systems for releasing biologically active agents form a field of research in continuous development. It is known that the administration of active ingredients to the animal or human body by different administration routes has difficulties. Some drugs, including peptides, proteins and polysaccharides, are not effectively absorbed through mucous surfaces given the limited permeability of epithelial barriers. For example, insulin, which is currently administered subcutaneously and is therefore undesirable for the patient, is one of those active ingredients with poor capacity to pass through mucous barriers such as nasal or intestinal mucous barriers, which makes it necessary to develop administration systems allowing a better absorption of this active molecule if alternative routes to subcutaneous administration are to be found.

The incorporation of active ingredients in small-sized particles is emphasized among the recently proposed possibilities to overcome the biological barriers faced by drugs. The interaction of said particles with mucous membranes is affected, among other factors, by the size of these particles, said interaction increasing with the decrease in the particle size.

Thus, patent application US2004138095 describes an aqueous nanoparticle suspension for releasing insulin, among other active ingredients, based on three-block polyethylene glycol / hydrophilic polyaminoacid / hydrophobic polyaminoacid copolymers. In turn, patent US 5,641,515 describes a pharmaceutical formulation for the controlled release of insulin, comprising nanoparticles formed by biodegradable polycyanoacrylate in which insulin is entrapped forming a complex.

Nanoparticle systems based on hydrophilic polymers have also been developed for their application as systems for releasing drugs. This is shown by the abundant literature existing in this field. Several works have been published which described several methods for preparing hydrophilic nanoparticles based on macromolecules with a natural origin, such as albumin nanoparticles (W. Lin et al., Pharm. Res., 11, 1994) and gelatin (H.J. Watzke et al., Adv. Colloid Interface Sci., 50, 1-14, 1994), and based on polysaccharides such as alginate (M. Rajaonarivonvy et al., J. Pharm. Sci., 82, 912-7, 1993). However, most of these methods require using organic solvents, oils and high temperatures, aspects which limit the exploitation of these systems enormously. The most innocuous of these methods is that proposed for preparing alginate nanoparticles, based on an ionic gelation process in the presence of calcium and subsequent hardening in the presence of the cationic polyelectrolyte poly-L-lysine. However, these nanoparticles have drawbacks relating to systemic toxicity and the high cost of poly-L-lysine.

An alternative to these systems has been the development of chitosan nanoparticles, there being publications in the state of the art that describe their usefulness for administering active ingredients as well as a process for obtaining them (J. Appl. Polym. Sci. 1997, 63, 125-132; Pharm. Res. 14, 1997b, 1431-6; Pharm. Res. 16, 1991a, 1576-81; S.T.P. Pharm. Sci. 9,1999b, 429-36, Pharm. Res. 16, 1999, 1830-5; J. Control Release 74, 2001, 317-23 and US5,843,509). The drawback of these nanoparticles is their limited stability in certain pH and ionic strength conditions.

Document WO-A-01/32751 relates to a process for preparing chitosans or chitosan derivatives in the form of nanoparticles, consisting of dissolving chitosan or the derivatives in an aqueous medium and subsequently raising the pH in the presence of a surface modifier to such an extent that chitosan is precipitated.

Document WO-A-99/47130 relates to nanoparticles having a biocompatible and biodegradable polyelectrolyte complex, from at least one polycation (which may be chitosan) and at least one polyanion, as well as an active ingredient, the nanoparticles being able to be obtained by additionally treating the polyelectrolyte complex during or after its formation with at least one crosslinking agent (glyoxal, TSTU or EDAP).

Obtaining chitosan nanoparticles combined with polyoxyethylene (ES 2098188 and ES 2114502), in addition to the active ingredient which may be a therapeutic or antigenic macromolecule, is also known. The formation of these nanoparticles occurs due to a joint precipitation process of chitosan and of the active macromolecule in the form of polymeric nanoaggregates, caused by the addition of a basic agent such as tripolyphosphate.

In addition, chitosan can be modified by the covalent bonding with polyethylene glycol through the amino function, which is known as pegylation. Patents EP 1304346 and US 6,730,735 describe a composition for administering drugs through mucous membranes, comprising a chitosan and PEG conjugate, both being covalently bonded through the chitosan amino group.

Patent application US2004/0156904 describes a system for releasing pharmaceutical agents, in which the active agent is incorporated to a matrix prepared from a composition including chitosan-PEG and a water-insoluble polymer such as poly(lactic-co-glycolic acid) (PLGA).

Patent application WO01/32751 describes the obtention of chitosan nanoparticles precipitating in the presence of a surfactant, including polyethylene glycol, when the pH of the solution in which they are located increases. However, PEG does not bond covalently to chitosan.

In spite of the many publications aimed at developing system for releasing drugs, there is still a need to provide a type of nanoparticle system having a great capacity for associating to a biologically active molecule and allowing its release at a controlled rate.

### BRIEF DESCRIPTION OF THE INVENTION

The inventors have found that a system formed by PEG-modified chitosan nanoparticles obtained by means of an ionic gelation process in the presence of an agent causing the crosslinking of chitosan, allows an effective association of biologically active molecules as well as their subsequent release in a suitable biological environment. PEG-modified chitosan nanoparticles have significant properties with respect to non-pegylated chitosan nanoparticles, for example in nasal insulin administration or in immunogenicity as a response to nasal diphtheria toxoid administration.

Thus, an object of the present invention is aimed at a system comprising nanoparticles for releasing a biologically active molecule, in which the nanoparticles comprise a conjugate comprising a) at least 50% by weight of chitosan or a derivative thereof and b) less than 50% by weight of polyethylene glycol (PEG) or a derivative thereof, where both components a) and b) are covalently bonded through the chitosan amino groups, and characterized in that said nanoparticles are crosslinked by means of a crosslinking agent.

The expression "biologically active molecule" has a broad meaning and comprises molecules such as low molecular weight drugs, polysaccharides, proteins, peptides, lipids, oligonucleotides, and nucleic acids and combinations thereof. In one variant of the invention, the function of the biologically active molecule is to prevent, palliate, cure or diagnose disease. In another variant of the invention, the biologically active molecule has a cosmetic function.

A second aspect of the present invention relates to a pharmaceutical composition or vaccine comprising the nanoparticles defined above. In a preferred aspect, the composition or vaccine is for mucosal administration.

In another aspect, the invention is aimed at a cosmetic composition comprising the nanoparticles defined above.

Another aspect of the present invention relates to a composition comprising the chitosan-PEG nanoparticles inside which one or more biologically active molecules, such as a drug, a vaccine or genetic material, can be retained. Peptides, proteins or polysaccharides which are not considered biologically active molecules *per se* but can contribute to the efficiency of the administration system can also be entrapped in the nanostructure.

A final aspect of the invention is formed by a process for obtaining a system for releasing a biologically active molecule as defined, comprising:
a) preparing an aqueous chitosan-PEG conjugate solution;
b) preparing an aqueous crosslinking agent solution; and
c) mixing, with stirring, the solutions of steps a) and b), such that chitosan-PEG nanoparticles are spontaneously obtained by means of ionic gelation and subsequent precipitation.

In one variant of the process, the active ingredient is incorporated either to the aqueous chitosan solution or to the aqueous crosslinking agent solution, before mixing both phases.

### DETAILED DESCRIPTION OF THE DRAWINGS

Figure 1A: Effect of the chitosan pegylation degree, of the pH of the polymer solution and of the chitosan-PEG/TPP ratio on the nanoparticle size.
Figure 1B: Effect of the chitosan pegylation degree, of the pH of the polymer solution and of the chitosan-PEG/TPP ratio on the polydispersity in the nanoparticle size.
Figure 2A: Agarose gel electrophoresis analysis of the plasma DNA associated to chitosan and chitosan-PEG nanoparticles after 1 day of incubation in acetate buffer (pH: 4 and 7.4) and in purified water (MQ).
Figure 2B: Agarose gel electrophoresis analysis of the plasma DNA associated to chitosan and chitosan-PEG nanoparticles after 4 weeks of incubation in acetate buffer (pH: 4 and 7.4) and in purified water (MQ).
Figure 3 Agarose gel electrophoresis analysis of the plasma DNA associated to chitosan and chitosan-PEG nanoparticles in the presence of chitosanase.
Figure 4A: Effect of the pegylation degree on the efficiency in the transfection of high molecular weight chitosan (CS) nanoparticles.
Figure 4B: Effect of the pDNA load on the efficiency in the transfection of high molecular weight chitosan (CS) nanoparticles.
Figure 5A: Effect of the pDNA load on the efficiency in the transfection of low molecular weight chitosan (CS) nanoparticles.
Figure 5B: Effect of the pegylation degree on the efficiency in the transfection of low molecular weight chitosan (CS) nanoparticles.
Figure 6: Blood sugar after the intranasal administration of 10 U/kg of insulin contained in different formulations or acetate buffer (control). Blood sugar is expressed in % with respect to the baseline values, in mean value ± S.E.M. The following values are the baseline values before the different administrations: acetate buffer (■): 423±16 mg/dl (n=7); insulin (□): 430±15 mg/dl (n=7); insulin in chitosan solution (•) : 439±12 mg/dl (n=8); chitosan nanoparticles (◆): 469±14 mg/dl (n=7); chitosan-PEG nanoparticles (o): 458±21 mg/dl (n=8).
Figure 7: Glucose tolerance tests carried out after the intranasal administration of the formulations containing insulin or acetate buffer in diabetic rats which have fasted overnight. Glucose (2 g per kg of body mass) was administered intragastrically at time 0 and one hour after the intranasal administrations: acetate buffer (■) (n=10); insulin (□) (n=6); insulin in chitosan solution (•) (n=6); chitosan nanoparticles (◆) (n=6); chitosan-PEG nanoparticles (○) (n=6).
Figure 8: Final titers of IgG anti-TD in mouse serum after the intranasal administration of 10 µg of TD incorporated in different chitosan and chitosan-PEG nanoparticles formulations on days 0, 7 and 14 (n=6). IN: Intranasal; IP: Intraperitoneal.
* Statistically significant differences (α<0.5).

### DETAILED DESCRIPTION OF THE INVENTION

The system of the present invention comprises nanoparticles, the structure of which comprises a crosslinked chitosan and polyethylene glycol (PEG) conjugate, into which an active ingredient can be incorporated.

The term "nanoparticle" is understood as a structure comprising a conjugate, result of the covalent bonding between chitosan and PEG through the chitosan amino groups, which conjugate is furthermore crosslinked by means of ionic gelation by the action of an anionic crosslinking agent. The formation of covalent bonds and the subsequent ionic crosslinking of the system generate independent and observable characteristic physical entities, the average size of which is less than 1 µm, i.e., an average size comprised between 1 and 999 nm.

"Average size" is understood as the average diameter of the population of nanoparticles moving together in the aqueous medium in which they are formed. The average size of these systems can be measured by means of standard processes known by any persons skilled in the art and which are described in the experimental part below, for example.

The nanoparticles of the system are characterized by having an average particle size of less than 1 µm, they preferably have an average size comprised between 1 and 999 nm, preferably between 50 and 800 nm, and still more preferably between 50 nm and 500 nm. The average particle size is mainly affected by the ratio of chitosan with respect to PEG, by the chitosan deacetylation degree and also by the particle formation conditions (chitosan-PEG concentration, crosslinking agent concentration and the ratio between both). The presence of PEG reduces the average particle size with respect to systems formed by non-pegylated chitosan.

In addition, the nanoparticles can have an electric charge (measured by means of the Z potential), the magnitude of which can range from +0.1 mV to +50 mV, preferably between +1 and +40 mV, depending on the mentioned variables and particularly on the functionalization degree of chitosan with PEG. The positive charge of the nanoparticles may be interesting for favoring the interaction thereof with mucous surfaces. Nevertheless, neutral charge can be more interesting for the parenteral administration thereof.

The system comprising nanoparticles for releasing a biologically active molecule defined above has a chitosan content in the conjugate of more than 50%, preferably more than 75% by weight. For its part, the PEG content in the conjugate is less than 50%, preferably less than 25%.

Chitosan is a natural polymer derived from chitin (poly-N-acetyl-D-glucosamine), in which an important part of the acetyl groups of the N have been eliminated by hydrolysis. The deacetylation degree is generally in a range comprised between 30 and 95%, preferably between 60 and 95%, which indicates that between 5 and 40% of the amino groups are acetylated. It therefore has an aminopolysaccharide structure and a cationic character. It comprises the repetition of monomeric units of formula (I): 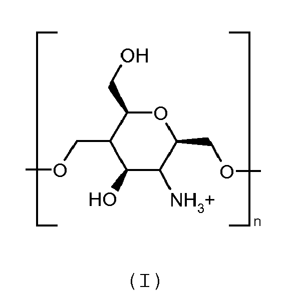 wherein n is an integer, and furthermore m units where the amino group is acetylated. The sum of n+m represents the polymerization degree, i.e. the number of monomeric units in the chitosan chain.

The chitosan used to obtain the chitosan-PEG conjugates of the present invention has a molecular weight comprised between 5 and 2000 kDa, preferably between 10 and 500 kDa, more preferably between 10 and 100 kDa. Examples of commercial chitosans which can be used are UPG 113, UP CL 213 and UP CL113, which can be obtained from NovaMatrix, Drammen, Norway.

The number of monomeric units comprising the chitosan used to obtain the chitosan-PEG conjugates is comprised between 30 and 3000 monomers, preferably between 60 and 600.

A chitosan derivative can also be used as an alternative to chitosan, understanding as such a chitosan in which one or more hydroxyl groups and/or one or more amino groups have been modified for the purpose of raising the solubility of chitosan or increasing the mucoadhesive character thereof. These derivatives include, among others, acetylated, alkylated or sulfonated chitosans, thiolated derivatives, as described in Roberts, Chitin Chemistry, Macmillan, 1992, 166. When a derivative is used, it is preferably selected from O-alkyl ethers, O-acyl esters, trimethyl chitosans, chitosans modified with polyethylene glycol, etc. Other possible derivatives are salts, such as citrate, nitrate, lactate, phosphate, glutamate, etc. In any case, persons skilled in the art know how to identify the modifications which can be carried out on chitosan without affecting the commercial viability and stability of the final formulation.

For its part, in its most usual form, polyethylene glycol (PEG) is a polymer of formula (II):

H-(O-CH₂-CH₂)ₚ-O-H (II)

wherein p is an integer representing the PEG polymerization degree. In the present invention, the PEG polymerization degree is in the range comprised between 50 and 500, which corresponds to a molecular weight between 2 and 20 kDa, preferably between 5 and 10 kDa.

A modified PEG in which one or the two terminal hydroxyl groups are modified is to be used to form the chitosan-PEG complex. The modified PEGs which can be used to obtain the chitosan-PEG conjugates include those having the formula (III):

X₁-(O-CH₂-CH₂)ₚ-O-X₂ (III)

wherein:
X₁ is a hydroxyl radical protecting group blocking the OH function for subsequent reactions. Hydroxyl protecting groups are well known in the art, representative protecting groups (already including the oxygen to be protected) are silyl ethers such as trimethylsilyl ether, triethylsilyl ether, tertbutyldimethylsilyl ether, tert-butyldiphenylsilyl ether, triisopropylsilyl ether, diethylisopropylsilyl ether, texyldimethylsilyl ether, triphenylsilyl ether, di-tert-butylmethylsilyl ether; alkyl ethers such as methyl ether, tert-butyl ether, benzyl ether, p-methoxybenzyl ether, 3,4-dimethoxybenzyl ether, trityl ether, allyl ether; alkoxymethyl ether such as methoxymethyl ether, 2-methoxyethoxymethyl ether, benzyloxymethyl ether, p-methoxybenzyloxymethyl ether, 2-(trimethylsilyl)ethoxymethyl ether; tetrahydropyranyl ether and related ethers; methylthiomethyl ether, esters such as acetate ester, benzoate ester; pivalate ester, methoxyacetate ester; chloroacetate ester; levulinate ester; carbonates such as benzyl carbonate, p-nitrobenzyl carbonate, tert-butyl carbonate, 2,2,2-trichloroethyl carbonate, 2-(trimethylsilyl)ethyl carbonate, allyl carbonate. Additional examples of hydroxyl protecting groups can be found in reference books such as "Protective Groups in Organic Synthesis" by Greene and Wuts, John Wiley & Sons, Inc., New York, 1999. In a preferred embodiment, the protecting group is an alkyl ether, more preferably it is methyl ether.
X₂ can be hydrogen or a bridge group allowing the anchoring to the chitosan amino groups. The preferred but not exclusive form among the bridge molecules used is a succinimide or a derivative thereof.

Alternatively, X₁ can also be a group allowing the anchoring with other groups other than the amino group. For example, maleimide is used as a bridge molecule to achieve the bonding with SH groups.

The number of chitosan amino groups reacting with PEG, or in other words, the functionalization of the chitosan amino groups with PEG, known as PEGylation, is comprised between 0.1% and 5%, preferably between 0.2% and 2%, more preferably between 0.5% and 1%.

The resulting chitosan-PEG conjugate has a molecular weight comprised between 5 and 3000 kDa, preferably between 10 and 500 kDa.

The nanoparticle system of the invention is characterized in that it has been formed by means of the ionic crosslinking of the chitosan-PEG conjugate. The crosslinking agent is an anionic salt allowing the crosslinking of the chitosan-PEG conjugate by means of ionic gelation, favoring the spontaneous formation of the nanoparticles. In the present invention, the crosslinking agent is a polyphosphate salt, the use of sodium tripolyphosphate (TPP) being preferable. The crosslinking to give rise to the nanoparticle system is simple and known to the person skilled in the art, as described in the background of the invention.

A second aspect of the present invention is formed by a pharmaceutical composition comprising the previously defined nanoparticles. Examples of pharmaceutical compositions include any liquid composition (nanoparticle suspension in water or in water with additives such as viscosifying agents, pH buffers, etc) or solid composition (lyophilized or atomized nanoparticles, forming a powder which can be used to prepare granulates, tablets or capsules) for their oral, buccal or sublingual administration or topical administration, or in liquid or semisolid form for their transdermal, ocular, nasal, vaginal or parenteral administration. In the case of non-parenteral administration routes, the contact of the nanoparticles with the skin or mucous membranes can be improved by providing the particles with a considerable positive charge, which will favor their interaction with the mentioned negatively charged surfaces. In the case of parenteral administration routes, more specifically for intravenous administration, these systems offer the possibility of modulating the *in vivo* distribution of the drugs or molecules associated thereto.

In a preferred aspect, the administration of the formulation is mucosal. The positive charge of the chitosan-PEG conjugate provides a better absorption of the drugs on the mucous surface through their interaction with the mucous membrane and the surfaces of the epithelial cells which are negatively charged.

The chitosan-PEG nanoparticles are systems having a high association capacity for bioactive molecules. This association capacity depends on the type of molecule incorporated as well as on the indicated formulation parameters. Therefore, another aspect of the present invention is formed by a composition comprising chitosan-PEG nanoparticles such as those defined previously and at least one biologically active molecule.

The term "biologically active molecule" relates to any substance which is used to treat, cure, prevent or diagnose a disease or which is used to improve the physical and mental wellbeing of humans and animals. These biologically active molecules can include from low molecular weight drugs to molecules of the type of polysaccharides, proteins, peptides, lipids, oligonucleotides and nucleic acids and combinations thereof. Examples of molecules associated to these nanoparticles include proteins such as tetanus toxoid and diphtheria toxoid, polysaccharides such as heparin, peptides such as insulin, as well as plasmids encoding several proteins.

In a preferred embodiment, the biologically active molecule is insulin. In another preferred embodiment, the biologically active molecule is the diphtheria or tetanus toxoid. In another preferred embodiment, the biologically active molecule is heparin. In another preferred embodiment, the biologically active molecule is a DNA plasmid.

The nanoparticle systems of the present invention can also incorporate other active molecules having no therapeutic effect but giving rise to cosmetic compositions. These cosmetic compositions include any liquid composition (nanoparticle suspension) or emulsion for their topical administration. The active molecules which can be incorporated to the nanoparticles include anti-acne agents, antifungal agents, antioxidants, deodorants, antiperspirants, anti-dandruff agents, skin whitening agents, tanning agents, UV light absorbers, enzymes, cosmetic biocides, among others.

Another aspect of the present invention is formed by a vaccine comprising the previously defined nanoparticles and an antigen. The administration of an antigen by the system formed by the nanoparticles allows achieving an immune response. The vaccine can comprise a protein, polysaccharide or can be a DNA vaccine. Strictly speaking, a DNA vaccine is a DNA molecule encoding the expression of an antigen giving rise to an immune response. In a preferred embodiment, the antigen is the tetanus toxoid and the diphtheria toxoid.

Another aspect of the present invention relates to a process for preparing chitosan-PEG nanoparticles such as those defined previously, comprising:
a) preparing an aqueous chitosan-PEG conjugate solution;
b) preparing an aqueous crosslinking agent solution; and
c) mixing, with stirring, the solutions of steps a) and b), such that chitosan-PEG nanoparticles are spontaneously obtained by means of ionic gelation and subsequent precipitation.

The pH of the initial chitosan-PEG conjugate solution is modified until reaching values comprised between 4.5 and 6.5 by means of adding sodium hydroxide prior to mixing both solutions.

In a variant of the process, the resulting chitosan-PEG/crosslinking agent ratio is comprised between 2/1 and 8/1, the 3/1 ratio being preferable, which ratio provides formulations with a relatively low polydispersity. Nevertheless, the use of higher chitosan-PEG/crosslinking agent ratio as well as the preparation of particles in more acidic media is also possible.

The presence of the crosslinking agent allows crosslinking the chitosan-PEG conjugate such that a mesh is formed, in which mesh a biologically active molecule which can later be released can be inserted. The crosslinking agent further confers to the nanoparticles the size, potential and structural characteristics making them suitable as a system for administering biologically active molecules.

The biologically active molecule can be directly incorporated to the solutions of steps a) or b), or in a prior dissolution in an aqueous or organic phase, such that the chitosan-PEG nanoparticles are spontaneously obtained containing the biologically active molecule by means of ionic gelation and subsequent precipitation.

The biologically active molecule can therefore be incorporated according to the following methods:
a) the active molecule is directly dissolved in the crosslinking agent or chitosan-PEG solutions;
b) the active molecule is dissolved in an acid or basic aqueous solution, prior to its incorporation to the crosslinking agent or chitosan-PEG solutions; or
c) the active molecule is dissolved in a water-miscible, polar organic solvent, prior to its incorporation to the crosslinking agent or chitosan-PEG solutions

The process for preparing chitosan-PEG nanoparticles can further comprise an additional step, in which said nanoparticles are lyophilized. From a pharmaceutical point of view, it is important to have the nanoparticles in lyophilized form because their stability during storage is thus improved. The chitosan-PEG nanoparticles (with different PEGylation degrees) can be lyophilized in the presence of a cryoprotector such as glucose at a 5% concentration. Other usual additives may be present. In fact, the determination of particle size before and after lyophilization is not significantly modified. In other words, the nanoparticles can be lyophilized and resuspended without causing a variation therein (Table I).

**Table I: Characteristics of the CS-PEG nanoparticles before and after lyophilization.**

| **Formulation** | **size (nm)** | **P.I.*** | **size (nm)** | **P.I.** |
|---|---|---|---|---|
| | **before lyophilization** | | **lyophilized with 5% glucose** | |
| chitosan-0.5% PEG | 74.7 ± 9.5 | 0.231 | 78.6 ± 18.3 | 0.224 |
| chitosan-1% PEG | 76.9 ± 6.0 | 0.500 | 87.7 ± 22.3 | 0.418 |

| | | | | |
|---|---|---|---|---|
| *P.I.: polydispersity index | | | | |

Several illustrative examples are described below which will show the features and advantages of the invention but which must not be interpreted as limiting the object of the invention.

### EXAMPLES

### Example 1

### Optimization in the preparation of chitosan-PEG nanoparticles

The chitosan-PEG nanoparticles were prepared according to the ionic gelation technique described for chitosan in WO 9804244, for example. Specifically, chitosan with a 0.5% or 1% pegylation degree (percentage of amino groups that are functionalized with PEG) was initially dissolved in ultrapure water at a concentration of 1 mg/mL. For the purpose of studying its possible effect on the formation of the nanoparticles, the initial pH of the chitosan-PEG solution was modified until reaching values comprised between 4.5 and 6.5 by means of adding NaOH before preparing the particles. Sodium tripolyphosphate (TPP) was also dissolved in water at different concentrations for the purpose of obtaining chitosan-PEG/TPP ratios of 2/1, 3/1 and 4/1. The formation of the nanoparticles occurs spontaneously after adding a fixed volume of TPP solution (0.6 mL) to a fixed volume of chitosan-PEG solution (1.5 mL) with magnetic stirring.

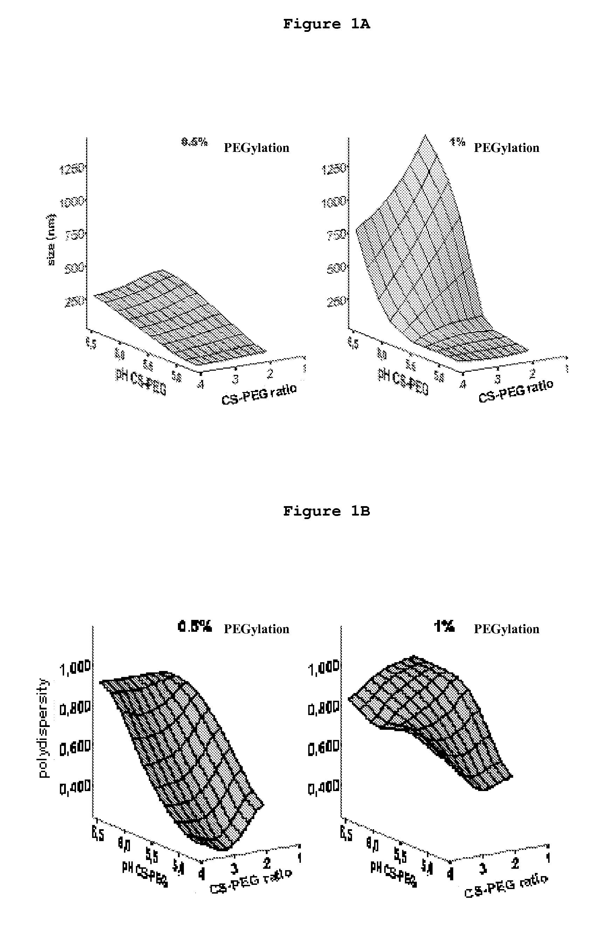

The effect caused by the chitosan pegylation degree, the pH of the polymer solution and the chitosan-PEG/TPP ratio on the nanoparticle size and polydispersity is shown in Figures 1A and 1B. Based on the results obtained, it can be concluded that the chitosan-PEG nanoparticles with a 0.5-1% pegylation degree can be easily prepared with a wide range of experimental conditions, the pH being the most influential parameter in the nanoparticle size. Moreover, the effect of the pH is more emphasized as the pegylation degree increases from 0.5 to 1%.

In addition, the nanoparticle size distribution is also affected by the chitosan pegylation degree as well as by the pH of the chitosan-PEG solution. The optimal chitosan-PEG/TPP ratio for obtaining formulations with a relatively low dispersity is apparently 3/1.

The nanoparticle size is determined by means of photon correlation spectroscopy, using a Zetasizer III (Malvern Instruments, Malvern, UK) for that purpose. The chitosan-PEG nanoparticle size ranged between 70 and 310 nm.

### Example 2

### Comparison between chitosan nanoparticles and chitosan-PEG nanoparticles

As can be observed in Table II, pegylation changes the solubility of chitosan. This affects the formation of the nanoparticles. The optimal chitosan/TPP ratio, typically located between 6/1 and 4/1, shifts to lower values, typically between 4/1 and 2/1, in the case of chitosan-PEG. These differences are probably due to the chemical modification of chitosan, undergoing a change of the available groups which can interact, being able to alter the ionotropic gelation conditions.

**Table II**

| | chitosan | chitosan-0.5% PEG | chitosan-1% PEG |
|---|---|---|---|
| initial pH | 4.7-4.8 | 4.6-4.7 | 4.6-4.7 |
| solubility limit | 6.4-6.5 | 6.9-7.1 | 7.1-7.2 |

As regards the nanoparticles, it can be observed that the nanoparticles prepared from pegylated chitosan are significantly different from those formed by pure chitosan. This is shown in Table III, showing the characteristics of the nanoparticles formed by chitosan, chitosan-PEG with 0.5% and 1% pegylation degrees (at the initial pH value, in its optimal polymer ratio).

Pegylation causes a marked decrease in nanoparticle size and in surface charge. In the latter case, the pegylation degree also has a strong influence because the surface charge decreases even more when the pegylation degree increases from 0.5% to 1%.

**Table III**

| | size (nm) | P.I. | potential (mV) |
|---|---|---|---|
| chitosan, 4/1 | 265 ± 10 | 0.363 | +29.1 ± 1.1 |
| chitosan-0.5% PEG, 3/1 | 74 ± 9 | 0.231 | +12.1 ± 1.8 |
| chitosan-1% PEG, 3/1 | 77 ± 6 | 0.500 | +1.6 ± 0.6 |

| | | | |
|---|---|---|---|
| P.I.=polydispersity index | | | |

### Example 3

### Formation and characterization of DNA-loaded chitosan nanoparticles with different pegylation degrees

In order to encapsulate it, plasmid DNA was incorporated to the TPP solution prior to the formation of the nanoparticles. This TPP solution containing the DNA was later added to the chitosan-PEG solution and was maintained with magnetic stirring. The theoretical DNA loads were 5, 10 or 20% with respect to the total amount of chitosan-PEG used to prepare the nanoparticles (1 mg). The efficiency of the encapsulation of plasmid DNA was always greater than 90%, as confirmed by fluorescence (Pico Green dsDNA dye) and agarose gel electrophoresis assays.

Tables IV, V and VI show the characteristics of the different chitosan and chitosan-PEG nanoparticles. Similarly to unloaded nanoparticles, DNA-loaded formulations containing PEG are much smaller and have less positive surface charge than the formulations without PEG.

In all the cases, the presence of DNA also causes changes in the characteristics of the carriers, especially at high DNA percentages, in which the surface charge generally decreases. As regards the nanoparticle size, large DNA loads allow inducing the formation of structures that are more crosslinked, which causes a decrease in the particle size. This can be observed in the case of non-pegylated carriers, in which the size decreases from approximately 270-300 nm to 220 nm.

However, the size of the pegylated carriers increases with the DNA load, especially when chitosan-PEG with a 0.5% pegylation degree is used.

**Table IV**

| chitosan without PEG, 4/1 | size (nm) | P.I. | potential (mV) |
|---|---|---|---|
| blank | 265±10 | 0.363 | 29.1±1.1 |
| +5% DNA | 278±17 | 0.340 | 40.5±5.6 |
| +10%DNA | 309±2 | 0.378 | 41.6±0.8 |
| +20%DNA | 216±7 | 0.363 | 35.2±1.8 |

**Table V**

| chitosan-0.5% PEG, 3/1 | size (nm) | P.I. | potential (mV) |
|---|---|---|---|
| blank | 74±9 | 0.231 | +121±1.8 |
| +5% DNA | 115±14 | 0.227 | +13.4±2.1 |
| +10%DNA | 131±13 | 0.207 | +11.3±3.9 |
| +20%DNA | 181±8 | 0.209 | +5.7±1.6 |

**Table VI**

| chitosan-1% PEG, 3/1 | size (nm) | P.I. | potential (mV) |
|---|---|---|---|
| blank | 77±6 | 0.500 | +1.6±0.6 |
| +5% DNA | 78±7 | 0.485 | +3.7±0.4 |
| +10%DNA | 78±10 | 0.256 | +1.3±2.1 |
| +20%DNA | 105±2 | 0.229 | -0.6±0.4 |

### Example 4

### In vitro plasmid DNA release

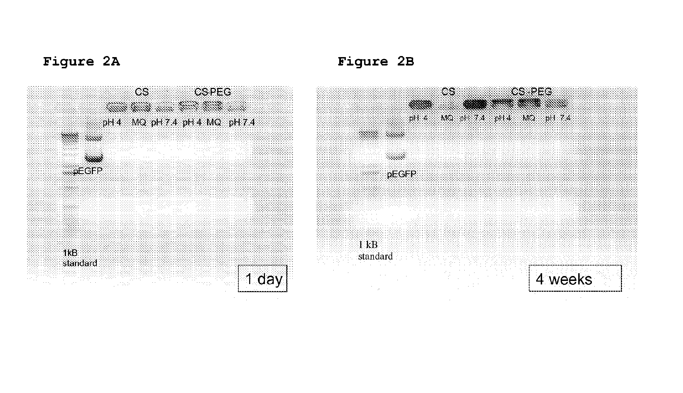

Plasmid DNA was effectively associated to both chitosan and chitosan-PEG particles. As shown in Figures 2A and 2B, plasmid DNA release is not detected (according to agarose gel electrophoresis analysis) when plasmid DNA-loaded nanoparticles are incubated for more than one month, both in acetate buffer (pH:4, pH:7.4) and in purified water (MQ).

### Example 5

### In vitro plasmid DNA release in the presence of enzymes

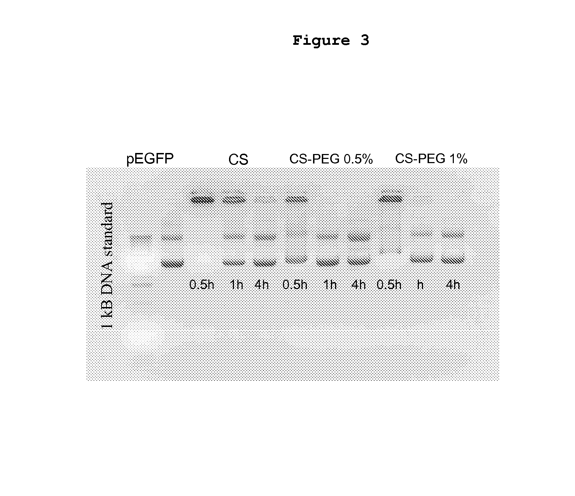

As can be observed from the electrophoresis analysis (Figure 3), plasmid DNA can be released from the chitosan and chitosan-PEG nanoparticles when plasmid DNA-loaded nanoparticles are incubated in acetate buffer at pH 6 in the presence of chitosanase (0.6 mg/mL).

These results show that plasmid DNA has been effectively associated to the nanoparticles, however, it is not irreversibly bound to them because it can be released when the polymeric carrier is enzymatically degraded.

### Example 6

### Efficiency in the transfection of (GFP) plasmid DNA associated to chitosan-PEG nanoparticles

The efficiency of the transfection by means of chitosan and chitosan-PEG nanoparticles has been evaluated for encoding the GFP plasmid in an HEK 293 cell line model.

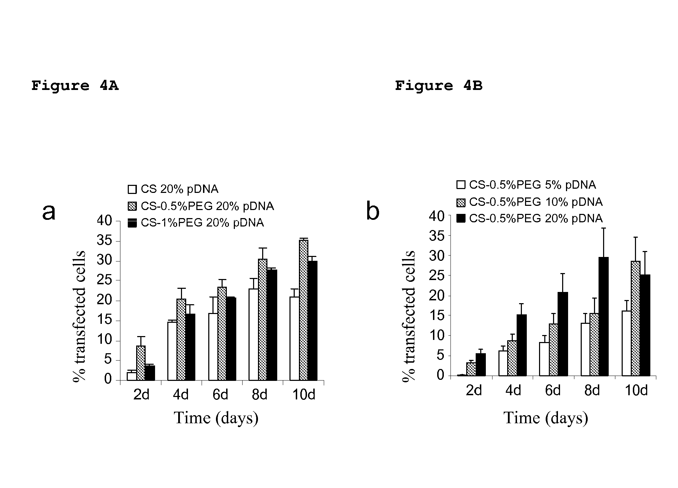

Figure 4 shows the effect caused by the chitosan pegylation degree (0.5% and 1%) on the efficiency in the transfection of high molecular weight chitosan nanoparticles (125 kDa, HMW CS NP) containing a 20% plasmid DNA (pDNA) load. The plasmid dose per well was 1 µg. The results indicate that a 0.5% pegylation degree has a positive effect on the transfection capacity of these nanoparticles. This pegylation degree was chosen for subsequent experiments. The results shown in Figure 4b indicate that the efficiency of the transfection increases with the pDNA load of the nanoparticles. This observation is interesting because the plasmid dose was constant (1 µg) and therefore the nanoparticle dose decreases as the pDNA load increases.

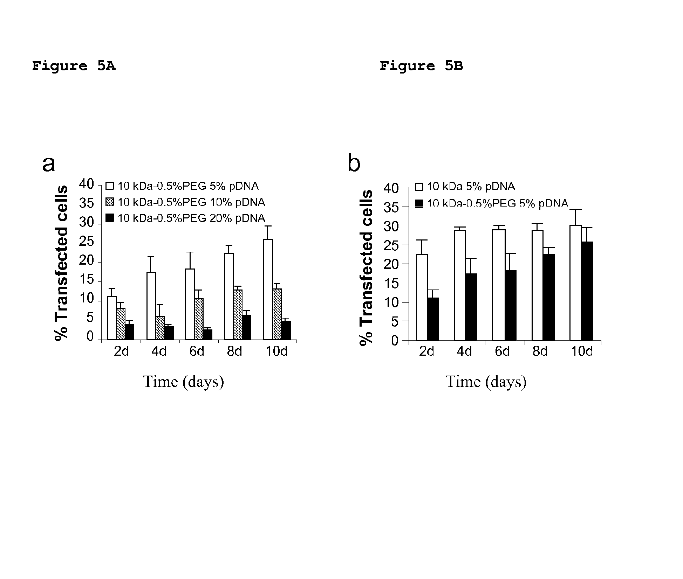

The effect of the plasmid load on the efficiency of the transfection was also evaluated for low molecular weight chitosan-PEG nanoparticles (10 kDa, LMW CS-PEG NP). In this case, the higher expression level was observed for the smaller load (5%) (Figure 5a). In addition, for this small load (5% pDNA), it was observed that pegylation had a negative effect on the efficiency of transfection (Figure 5b).

The main conclusion of these experiments is that chitosan pegylation has a positive (HMW CS-PEG NP) or negative (LMW CS-PEG NP) effect depending on the molecular weight of chitosan.

### Example 7

### Biological effect of the intranasal administration of encapsulated insulin and free insulin at a concentration of 10 U/kg

To encapsulate insulin in the chitosan nanoparticles, 2.4 mg of insulin were previously dissolved in an NaOH solution to which 1.2 mL of TPP were later added. This mixture was later added to 3 mL of chitosan. After 5 min with magnetic stirring, the preparation was centrifuged at 10,000 g for 40 min. The supernatant was removed and the precipitate was dissolved in a buffer. For its part, the same process was carried out to prepare the chitosan-PEG nanoparticles, but adding 10 mg/mL of PEG 400 to 2 mg/mL of chitosan.

The final size of the chitosan nanoparticles, determined by means of photon correlation spectroscopy, was 605±15 nm, whereas that of the chitosan-PEG nanoparticles was 590±6 nm.

Diabetes was initially induced in male Wistar rats weighing between 200 and 220 g by means of a streptozotocin injection (65 mg/kg i.v.) in a citrate buffer at pH 4.5. The rats were considered diabetic when the blood sugar concentration was greater than 400 mg/dl after three weeks of the treatment with streptozotocin.

Fasting rats were used to carry out this experiment. They were divided into different groups depending on the formulation which was to be administered to them. The different formulations consisted of: a control solution (acetate buffer, pH 4.3), insulin dissolved in acetate buffer, insulin dissolved in chitosan solution and insulin associated to chitosan nanoparticles and insulin associated to chitosan-PEG nanoparticles), the insulin concentration being of 10 U per kg of body mass. These formulations were administered by placing a small volume thereof (10-20 µl) in each nasal orifice using an Eppendorf pipette, the rats being anesthetized with ether in a supine position. The animals were then kept conscious during the entire experiment for the purpose of preventing any influence that the anesthesia might have on the blood glucose levels.

To determine the blood glucose levels, blood samples were collected from the tail vein before the nasal administration and every quarter of an hour until 90 minutes were completed. Subsequently they were collected every hour for 2 to 7 hours and finally 24 hours after the nasal administration. The blood glucose level was immediately determined using a glucose analyzer (Prestige from Chronolyss). The rats were kept fasting during the experiment.

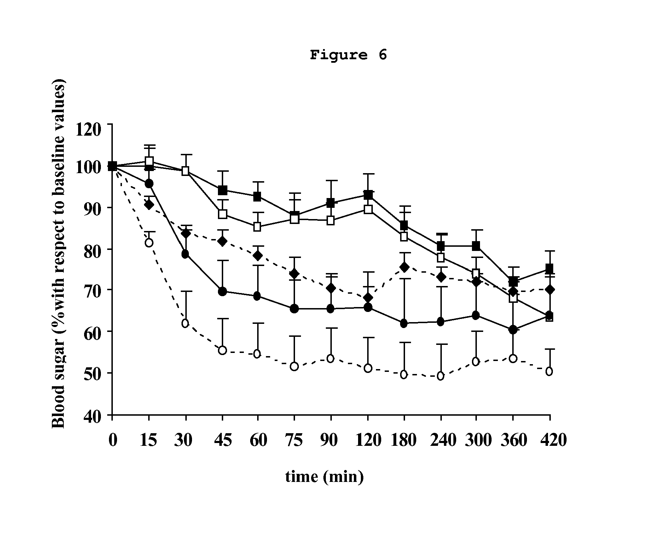

As shown in Figure 6, the nasal administration of acetate buffer, pH 4.3, causes the slow and progressive decrease of blood sugar according to time, the maximum decrease being 28% with respect to the control value 6 hours after the administration. The nasal administration of 10 U/kg of insulin in acetate buffer solution did not significantly change the previous result. However, when insulin is associated to chitosan-PEG nanoparticles, blood sugar levels decrease by 18% (p<0.01) within only 15 minutes after the administration, reaching a maximum decrease of 45-50% (p<0.01 and p<0.001 respectively) 45 minutes after said administration. It must be emphasized that this decrease is maintained after at least 7 hours have elapsed from the nasal administration. A marked decrease in blood sugar levels is also observed when insulin associated to chitosan nanoparticles is administered intranasally. In this case, the blood sugar decreases significantly starting from 30 minutes, specifically by 16% (p<0.01), the maximum decrease being 32% (p<0.001), observed 2 hours after the intranasal administration. Blood sugar also decreases when insulin is dissolved in a chitosan solution, but to a significantly lesser extent than when insulin is associated to chitosan-PEG nanoparticles.

### Example 8

### Effect of the nanoparticles containing insulin on the glycemic response to an oral glucose administration.

Diabetic rats which had fasted overnight were used to carry out this experiment. Each of the preparations described in Example 7 was nasally administered to them. After one hour, each group of rats received an oral glucose administration of 2 g per each kg of body mass. The blood sugar was measured in blood from samples collected from the tail vein before the glucose administration and after 10, 20, 30, 60, 90 and 120 minutes.

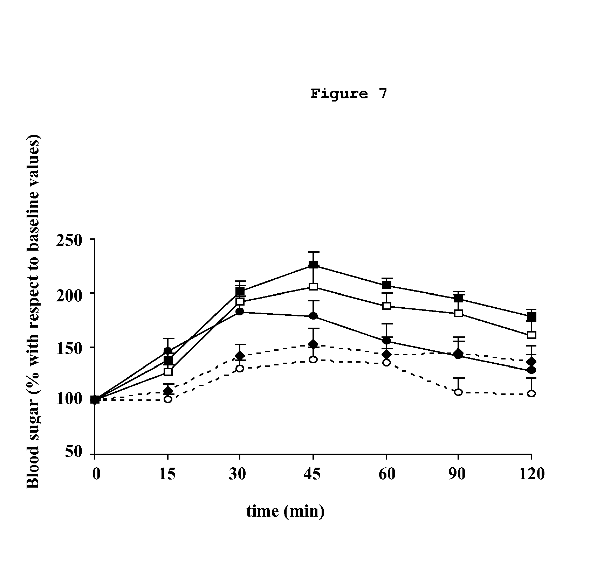

Figure 7 shows the effect of the nanoparticles containing insulin on the glycemic response to the oral glucose administration. In control rats which had received acetate buffer, the oral glucose administration was followed by an increase of blood sugar, the maximum value of which, 105% (p<0.001), was reached 30 minutes later. The blood sugar subsequently decreased gradually for 2 hours. For its part, the insulin dissolved in acetate buffer (10 U/kg) did not significantly change this result.

However, the same amount of insulin in a chitosan solution increased the glycemic response to glucose by 189% (p<0.05), whereas the insulin associated to chitosan or chitosan-PEG nanoparticles increased it by 193% (p<0.01) and 225% (p<0.01) respectively.

Therefore, comparing the A.U.C. (area under the curve) based on the different formulations analyzed, the most effective preparation was the preparation corresponding to insulin associated to chitosan-PEG nanoparticles, followed by insulin associated to chitosan nanoparticles and finally the preparation corresponding to insulin in a chitosan solution.

These results show that the use of the chitosan-PEG nanoparticles described in the present invention increase the nasal absorption of insulin in diabetic rats. The experimental results show that insulin (10 U/kg of body mass) associated to chitosan-PEG nanoparticles considerably reduced blood sugar starting from 15 minutes until at least 7 hours after the intranasal release and improves the glycemic response when an oral glucose load occurs. A less marked effect is detected with 4 U/kg of insulin associated to said nanoparticles.

When insulin is associated to nanoparticles formed only by chitosan or when insulin is in solution with chitosan, the efficiency is lower than when it is associated to chitosan-PEG nanoparticles, whereas free insulin does not have any significant effect on biological parameters after its intranasal release in diabetic rats.

### Example 9

### In vivo evaluation of the immune response caused by diphtheria toxoid-loaded nanoparticles

The association of diphtheria toxoid (DT) to the chitosan or chitosan-PEG nanoparticles is carried out by incorporating the DT to an aqueous TPP solution (300 µg of DT). The nanoparticles are formed spontaneously with the addition of different volumes of the aqueous TPP solution (1 mg/mL) to 3 mL of a chitosan or chitosan-PEG solution (1 mg/mL) with magnetic stirring. The volumes of the TPP solution were calculated for the purpose of achieving chitosan:TPP ratios of 8:1 and 2:1. Chitosan is marketed as its hydrochloride salt, Protosan Cl^{®} 113 and Protosan Cl^{®} 213 with an 86% deacetylation degree. The chitosan nanoparticles were isolated by centrifugation at 10,000 g for 40 minutes at 5°C. In the case of chitosan-PEG, the nanoparticles are collected on a centrifuge ultrafilter (Amicon^{®} ultra-4 100000 NMWL, Millipore) at 3800 g for 30 minutes. The supernatant was eliminated and the nanoparticles were resuspended in phosphate buffered saline with pH 7.4 for their administration in mice. The immunogenicity of the chitosan and chitosan-PEG formulations was carried out by means of intranasal immunization. Male BALB/c mice of 6 weeks of age and a weight of 22-25 g were used. The mice were divided into 5 groups. Two groups were treated with DT-loaded chitosan nanoparticles (CS-113 C1, CS-213 C1) and one group was treated with DT-loaded chitosan-PEG nanoparticles. The dose used was 10 µg of DT incorporated in 100 µg of nanoparticles and taken to 10 µL of phosphate buffer with pH 7.4, 5 µL being administered in each nasal orifice. Another group was treated with free toxoid (10 µg/mouse) in phosphate buffered saline with pH 7.4. Furthermore, as a control, one group received a DTP (diphtheria, tetanus and pertussis) vaccine intraperitoneally, adsorbed in aluminium phosphate (10 µg/mouse). The doses were administered on days 0, 7 and 14 to conscious rats.

Blood samples were taken from the tail of the mice 14, 28, 42, 56 and 70 days after the administration of the first dose to carry out the *in vivo* immune response assays. In turn, intestinal, bronchoalveolar and saliva wash samples were also collected on day 70. Salivation was induced by means of injecting pilocarpine (50 µL, 1 mg/mL) intraperitoneally. A 100 µL aliquot of the initial saliva flow of each mouse was collected. The mice were then anesthetized with pentobarbital and sacrificed. The bronchoalveolar washes were obtained by injecting and aspirating 5 mL of the washing medium in the trachea to inflate the lungs by means of an intravenous cannula. In turn, the intestinal segments (duodenum, jejunum, ileum) were removed aseptically and homogenized in 4 mL of a solution of 1 mM PMSF, 1 mM iodoacetic acid and 10 mM EDTA. The samples were clarified by means of centrifugation and sodium azide, PMSF and bovine serum were added as preservative. All the samples were stored at -20°C until carrying out the antibody concentration assays.

The evaluation of the responses of the antibody in serum and in mucous tissue was carried out by means of an ELISA test. The microplates (DYNEX, immulon®) were first coated with 100 µL of DT (4µg/well) in 0.05 M of carbonate buffer with pH 9.6 and incubated overnight at 4°C. Between steps, the wells were washed three times with PBST with pH 7.4 (0.01 M PBS, or phosphate buffer containing 5% v/v of Tween® 20). For the purpose of minimizing non-specific reactions, 100 µL of PBSTM (PBST containing 5% w/v of skimmed milk powder and 0.1% w/v of sodium azide as a preservative) were added to all the wells and they were incubated for 1 hour at 37°C. After washing with PBST the samples were diluted in series in two steps in PBSTM and the plates were incubated for another two hours at 37°C. Subsequently, 100 µL of goat anti-mouse IgG immunoglobulin peroxidase conjugate were diluted in 1:2000 in PBSTM, added to the wells, and incubated at 37°C for 2 h. The plates were washed and 50 µL of o-phenylenediamine dihydrochloride (0.45 mg/mL) were added in 0.05 M of citrate-phosphate buffer with pH 5.0 as a substrate. Following the color development (30 minutes at 37°C), the plates were read at 450 nm on a microplate reader (3350-UV, Biorad).

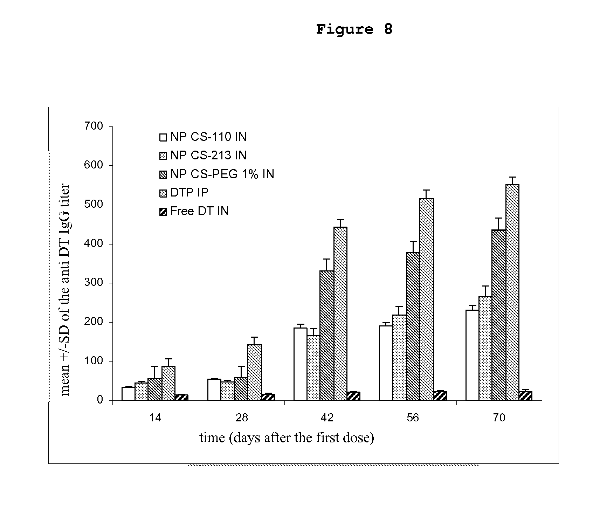

The anti-diphtheria IgG levels caused by the DT-loaded nanoparticles and the control DT solution following an intranasal immunization are shown in Figure 8. This figure also shows the results corresponding to the commercial formulation (DT absorbed in aluminium phosphate) administered intraperitoneally. In general terms, the results indicate that, after the first month, the IgG levels observed for DT-loaded nanoparticles were significantly better than those corresponding to the fluid vaccine (p<0.05). In fact, these values can be compared to those obtained for the formulation used as an adjuvant (DT adsorbed in aluminium phosphate) administered parenterally. Consequently, these results clearly show the adjuvant effect of the formulations containing the nanoparticles. Another observation to be emphasized is the increasing and lasting immune response over time. Finally, with respect to the influence of the nanoparticle composition, it is interesting to indicate that the molecular weight of chitosan has no effect on the immune response reached by the chitosan nanoparticles, however, chitosan PEGylation has a marked consequence on the efficiency of the nanoparticles. In fact, after one month, the IgG levels were significantly greater for the chitosan-PEG nanoparticles than for the chitosan nanoparticles.

## Claims

1. A system comprising nanoparticles for the release of biologically active molecules, wherein the nanoparticles comprise a conjugate comprising a) at least 50% by weight of chitosan or a derivative thereof and b) less than 50% by weight of polyethylene glycol (PEG) or a derivative thereof, wherein both components a) and b) are covalently bound through the chitosan amino groups, and **characterized in that** said nanoparticles are crosslinked by means of a crosslinking agent.

2. System according to claim 1, wherein the proportion of chitosan or a derivative thereof with respect to polyethylene glycol is preferably greater than 75% by weight.

3. System according to claim 1, wherein the proportion of polyethylene glycol is preferably less than 25% by weight.

4. System according to claims 1 to 3, wherein the chitosan polymerization degree or number of monomeric units comprising chitosan or a derivative thereof is comprised between 30 and 3000, preferably between 60 and 600.

5. System according to claims 1 to 4, wherein the chitosan or its derivative has a molecular weight comprised between 5 and 2000 kDa, preferably between 10 and 500 kDa, more preferably between 10 and 100 kDa.

6. System according to claims 1 to 5, wherein the chitosan or its derivative has a deacetylation degree comprised between 30% and 95%, preferably between 60% and 95%.

7. System according to claims 1 to 6, wherein the PEG is a modified PEG having a formula (III):
X₁-(O-CH₂-CH₂)ₚ-O-X₂
wherein X₁ is a hydroxyl radical protecting group, X₂ is a hydrogen or a bridge group allowing the anchoring to the chitosan amino groups, and p is the degree of polymerization.

8. System according to claim 7, wherein X₁ is an alkyl group, preferably methyl.

9. System according to claims 1 to 8, wherein the PEG has a degree of polymerization comprised between 50 and 500.

10. System according to claims 1 to 9, wherein the PEG has a molecular weight comprised between 2 and 20 kDa, preferably between 5 and 10 kDa.

11. System according to claims 1 to 10, wherein the functionalization of the chitosan amino groups or the amino groups of the chitosan derivative with PEG is comprised between 0.1% and 5%, preferably between 0.5% and 1%.

12. A system according to any of claims 1 to 11, further comprising a biologically active molecule selected from the group consisting of low molecular weight drugs, polysaccharides, proteins, peptides, lipids, oligonucleotides and nucleic acids and combinations thereof.

13. System according to any of claims 1 to 12, wherein the crosslinking agent is a polyphosphate salt, preferably sodium tripolyphosphate.

14. System according to any of claims 1 to 13, wherein the average nanoparticle size is comprised between 1 and 999 nanometers, preferably between 50 and 800 nm.

15. System according to any of claims 1 to 14, wherein the electric charge (Z potential) is comprised between +0.1 mV and +50 mV, preferably between +1 and +40 mV.

16. A pharmaceutical composition comprising a system such as that defined in any of claims 1 to 15 and a biologically active molecule capable of preventing, palliating or curing diseases.

17. Composition according to claim 16 for oral, buccal, sublingual, topical, transdermal, ocular, nasal, vaginal or parenteral administration.

18. Composition according to claim 16 or 17, wherein the biologically active molecule is selected from polysaccharides, proteins, peptides, lipids, oligonucleotides, nucleic acids and combinations thereof.

19. Composition according to any of claims 16 to 18, wherein the biologically active molecule is insulin, heparin, protein antigens or DNA plasmids.

20. Cosmetic composition comprising a system for releasing a biologically active molecule such as that defined in any of claims 1 to 15.

21. Cosmetic composition according to claim 20, wherein the active molecule is selected from anti-acne agents, antifungal agents, antioxidants, deodorants, antiperspirants, anti-dandruff agents, skin whitening agents, tanning agents, UV light absorbers, enzymes and cosmetic biocides.

22. A vaccine comprising a system for the release of a biologically active molecule such as that defined in any of claims 1 to 15 and an antigen.

23. Vaccine according to claim 22, wherein the antigen is selected from proteins, polysaccharides and DNA molecules.

24. Vaccine according to claim 22 or 23, wherein the antigen is the tetanus toxoid or the diphtheria toxoid.

25. Process for obtaining a system for the controlled release of a biologically active molecule according to any of claims 1 to 15 comprising:
a) preparing an aqueous chitosan-PEG conjugate solution;
b) preparing an aqueous crosslinking agent solution; and
c) mixing, with stirring, the solutions of steps a) and b), such that chitosan-PEG nanoparticles are spontaneously obtained by means of ionic gelation and subsequent precipitation.

26. Process for obtaining nanoparticles according to claim 25, wherein the crosslinking agent is a tripolyphosphate, preferably sodium tripolyphosphate.

27. Process according to any of claims 25 and 26, wherein the biologically active molecule is previously dissolved in a) or in b) or in another aqueous or organic phase which is added to a) or b).

28. Process according to claim 27, wherein the biologically active molecule is selected from insulin, heparin, DNA plasmid, tetanus toxoid and diphtheria toxoid.
